Europäisches Patentamt

European Patent Office

Office européen des brevets

(11) Publication number : **0 519 721 A1**

# EUROPEAN PATENT APPLICATION

(21) Application number : **92305605.5**

(51) Int. Cl.⁵ : **A61B 17/34**

(22) Date of filing : **18.06.92**

(30) Priority : **18.06.91 GB 9113102**

(43) Date of publication of application :
**23.12.92 Bulletin 92/52**

(84) Designated Contracting States :
**AT BE CH DE DK ES FR GB GR IT LI LU MC NL PT SE**

(71) Applicant : **Rocket of London Limited**
**Imperial Way**
**Watford, Hertfordshire WD2 4XX (GB)**

(72) Inventor : **Tyrrell, James Nicholas**
**8 Freesia Drive**
**Bisley, Surrey GU24 9HA (GB)**
Inventor : **Robson, Richard**
**6 Cuillin Close, Lambton**
**Washington, Tyne & Wear NE38 0PU (GB)**

(74) Representative : **Pattullo, Norman et al**
**Murgitroyd and Company 373 Scotland Street**
**Glasgow G5 8QA (GB)**

(54) **A lock device.**

(57)   A lock device for use for example in a pneumoperitineal needle (1) comprises a housing (8), a first lock member (15) mounted within the housing, a second lock member (5) movably mounted in the housing for movement between an unlocked position, a primed position and a locked position, and a helical spring (10) to bias the second lock member from the primed position towards the locked position, at least one of the first and second members being deformable to permit engagement of lock members being deformable to permit engagement of the first and second lock members, when the second lock member moves from the primed position to the locked position under the action of the helical spring to lock the lock members to each other.

_FIG.4_

EP 0 519 721 A1

This invention relates to a lock device and in particular, but not exclusively, a lock device for use with a surgical instrument, such as a pneumoperitoneal needle.

Pneumoperitoneal needles, and especially needles of the Verres type, are generally used to facilitate endoscopic examination of the abdominal cavity. The needle is inserted through the external wall and into the abdominal cavity which is then insufflated with a suitable gas through the needle before the endoscope is inserted. The Verres type pneumoperitoneal needles have a spring loaded blunt obturator which is mounted inside a large diameter hollow needle. During insertion of the needle through the abdominal wall the resistance against the end of the pneumoperitoneal needle is sufficient to force the obturator to retract within the hollow needle which enables the sharp tip of the needle to penetrate the abdominal wall. Once the pneumoperitoneal needle has penetrated the abdominal wall and entered the abdominal cavity the resistance against the blunt end of the obturator decreases sufficiently to allow the spring to push the blunt end of the obturator forward so that it extends beyond the sharp tip of the needle.

As the blunt end of the obturator extends-beyond the sharp tip of the needle it helps to prevent puncture or laceration of the intra-abdominal structures by the sharp tip of the needle. However, because the spring used with the obturator is relatively weak to enable the obturator to be retracted during insertion through the abdominal wall, the obturator may be accidentally retracted by the pressure of the intra-abdominal structures such as the visceral organs which may cause accidental trauma to the organs.

In accordance with the present invention, a lock device comprises a housing, a first lock member mounted within the housing, a second lock member movably mounted in the housing for movement between an unlocked position, a primed position and a locked position, and biassing means coupled between the housing and the second lock member to bias the second lock member from the primed position towards the locked position, at least one of the first and second lock members being deformable to permit engagement of the first and second lock members, when the second lock member moves from the primed position to the locked position under the action of the biassing means, to lock the lock members to each other.

The invention provides a lock device which may be used with a surgical instrument, such as a pneumoperitoneal needle to maintain the obturator in its extended position after the pneumoperitoneal needle has penetrated a body cavity such as the abdomen.

Preferably, the lock members may be released from engagement with each other by applying a predetermined minimum force to one of the lock members to move the second lock member to the unlocked position.

Typically, the second lock member is linearly movable in the housing and preferably, the unlocked position is located between the primed position and the locked position.

Preferably, the second lock member is biassed towards the locked position by the biassing means and typically, the biassing means may be provided by a spring such as a helical spring.

Preferably, the other lock member has a tapered profile which typically, assists the deformation of the other lock member to permit engagement of the first and second lock members.

Preferably, the tapered profile is provided by the other of the first and second members comprising a frustoconical shape. Preferably, the deformable lock member is provided by a deformable flange which engages with a recess in the other lock member. In the preferred embodiment the first lock member is deformable.

Preferably, the biassing means requires a compression force of approximately 1.52 Newtons in order to move the second lock member to the primed position and typically, the predetermined minimum force is greater than 4.9 Newtons and is preferably, at least 7.36 newtons. In the preferred embodiment, the distance between the primed position and locked position is approximately 17mm and the distance between the unlocked position and the primed position is approximately 14mm.

Typically, the lock device is incorporated into a surgical instrument, such as a pneumoperitoneal needle. Typically, the surgical instrument comprises a needle with a movable obturator mounted therein and typically, the second lock member is coupled to the obturator and the needle is coupled to a handle which provides the housing for the lock device. Preferably, the obturator extends beyond the tip of the needle when the second lock member is in the unlocked position and when the second lock member is in the locked position. Typically, the obturator is retracted within the needle when the second lock member is in the primed position.

Preferably, an air passageway is provided through the lock device and the obturator is hollow to enable an insufflating gas to be passed through the lock device and the obturator in order to introduce gas into a body cavity into which the surgical instrument is inserted in use. In the preferred embodiment, the surgical instrument is provided with a coupling, which enables attachment of a luer lock connector to enable connection of a gas supply which is typically carbon dioxide. Preferably, the lock device permits a flow rate of carbon dioxide of at least 3000 millilitres per minute at a pressure of 10 psi and a temperature of 20°C and preferably the device has a back pressure which is less than or equal to approximately 14 millimetres Hg.

An example of a lock device in accordance with the invention will now be described with reference to the accompanying drawings in which:-

Fig.1 is a plan view of a verres type pneumoperitoneal needle;

Fig. 2 is a cross-sectional view through a handle of the needle shown in Fig. 1 which schematically shows a lock device in an unlocked position;

Fig. 3 is a schematic cross-sectional view through the handle showing the lock device in a primed position; and,

Fig. 4 is a schematic cross-sectional view through the handle showing the lock device in a locked position.

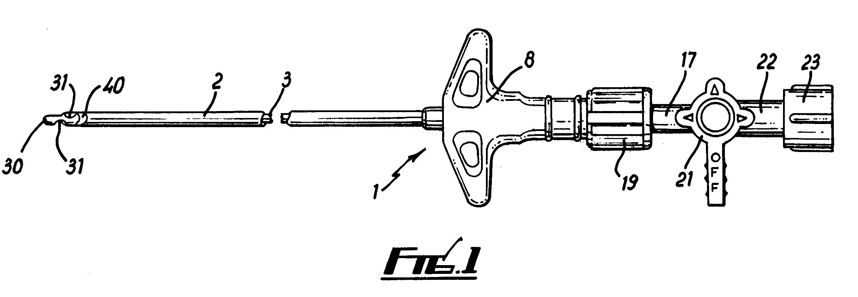

Fig. 1 shows a pneumoperitoneal needle 1 which is of the Verres type and which comprises a hollow needle body 2 with a tip 40 which is connected at its other end to a handle 8. Within the needle body 2 is located an obturator 3 which has a blunt end 30 and apertures 31 adjacent to a tip 30. The obturator 3 is hollow and is connected to a gas supply (not shown) by means of a passageway within the handle 8 and tubing 17, 22 which is connected to the needle 1 by a luer lock connector 19 and to the gas supply by a connector 23. The tubing 17, 22 is separated by a on/off valve 21 which may be used to isolate the needle 1 from the gas supply.

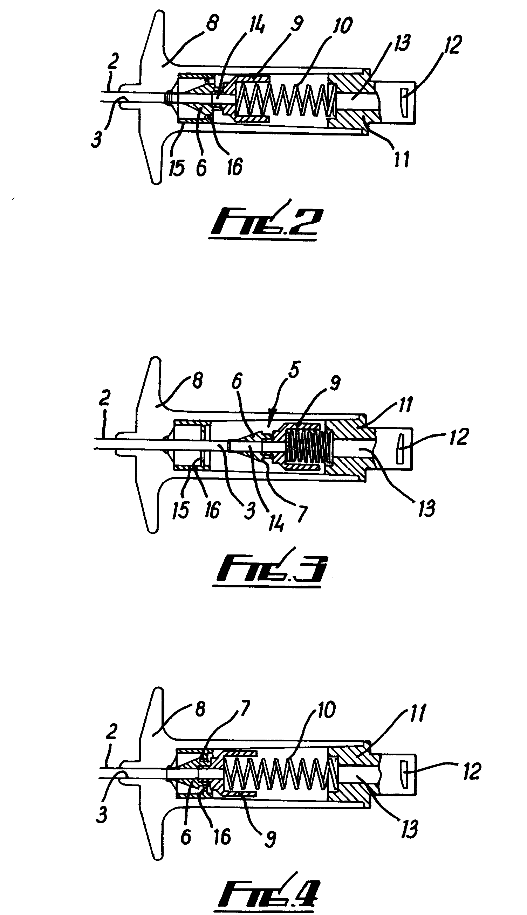

As shown in more detail in Figs. 2 to 4 the handle 8 is hollow and is fixed to the end of the needle body 2. Slidably mounted within the needle body 2 is the obturator 3 which is connected to a movable lock member 5 which has a frustoconical section 6 provided with a flange 7. The frustoconical section 6 is connected to a spring retainer portion 9 in which the end of a helical spring 10 is located. The opposite end of the helical spring 10 is retained within an end cap 11 which is inserted into the end of the hollow handle 8.

The end cap 11 has a connecting profile 12 moulded on to it for attachment to a luer connection 19. The end cap 11 also has a passageway 13 passing through it and the movable lock member 5 is also provided with a passageway 14. Mounted within the hollow handle 8 is a stationery lock member 15 which has a deformable flange 16 extending radially inward from the body of the stationary lock member 15.

In use, the pneumoperitoneal needle 1 described above is inserted into the abdominal cavity of a patient by pressing the blunt end 30 of the obturator against the skin of the patient. The pressure exerted on the handle 8 is transferred through the end cap 11, and spring 10 to the obturator 3 and this causes the spring 10 to compress from the position shown in Fig. 2 to the position shown in Fig. 3. When the movable lock member 5 is in the position shown in Fig. 3 the blunt end 30 of the obturator 3 is retracted within the needle body 2 and the tip 40 of the needle is exposed so that pressure exerted by an operator may cause the tip 40 to penetrate the abdominal wall of the patient so that the needle body 2 with obturator 3 therein penetrates through the abdominal wall of the patient so that the tip 40 of the needle body 2 enters the abdominal cavity.

When the tip 40 has entered the abdominal cavity the pressure is removed from the end 30 of the obturator 3 and the movable lock member 5 is forced away from the end cap 11 by the helical spring 10 and towards the stationary lock member 15. The extension force of the spring 10 is sufficient for the frustoconical section 6 to abut the deformable flange 16 and cause the flange 16 to deform so that the flange 7 of the frustoconical section 6 passes through the flange 16 to the position shown in Fig. 4.

In the position shown in Fig. 4 the obturator 3 extends beyond the tip 40 of the needle body 2 and the blunt end 30 protects the intra-cavity structures within the abdominal cavity of the patient from damage by the tip 40. The flange 16 is chosen so that it deforms sufficiently to enable the spring 10 to push the movable lock member 5 to the position shown in Fig. 4 but is sufficiently rigid to prevent any of the intra-cavity structures of the abdomen of the patient exerting enough force on the end 30 of the obturator to cause the flange 16 to deform and the obturator to retract within the needle body 2.

After the needle body 2 has been inserted into the abdominal cavity of a patient the valve 21 may be turned to the on position to allow an insufflating gas, such as carbon dioxide to pass through the tubing It, through the passageways 13, 14, through the inside of the hollow obturator 3 and exit into the abdominal cavity through the apertures 31 in the obturator 3 to insufflate the abdominal cavity of the patient.

After use the pneumoperitoneal needle 1 is removed from the body cavity and the obturator 3 and movable lock member 5 may be reset to the unlocked position shown in Fig. 2 by pressing the blunt end 30 of the obturator 3 against a sterile surface with sufficient force to deform the flange 16 and cause the movable lock member 5 to move to the position shown in Fig. 2.

Preferably, the spring 10 has a compression force of approximately 1.52 Newtons plus or minus 0.05 Newtons and when the movable lock member 5 is in the locked position shown in Fig. 4 then the deformability of the flange 16 is chosen so that a force of at least 4.9 Newtons plus or minus 0.1 Newtons may be exerted on the end of the obturator 3 without the lock member 5 moving to the position shown in Fig. 2 and the obturator 3 retracting into the needle body 2. Preferably, for the application described above the deformable flange 16 is chosen so that a force in excess of approximately 7.36 Newtons is required on the end of the obturator 3 in order to move the lock member 5 from the position shown in Fig. 4 to the position shown in Fig. 2.

An advantage of the lock device disclosed above is that it cannot be accidentally reset by an operator when the needle body 2 is inserted into a body cavity. In addition, the mechanism is relatively simple and does not require a large number of moving parts which would increase the likelihood of failure of the device. The relatively simply construction of the device has the advantage of enabling the device to be produced relatively inexpensively and hence, the possibility of the device being a disposable one time use product. This has the advantage of not requiring the instrument to be sterilised between uses.

Modifications and improvements may be incorporated without departing from the scope of the invention.

## Claims

1. A lock device comprising a housing, a first lock member mounted within the housing, a second lock member movably mounted in the housing for movement between an unlocked position, a primed position and a locked position, and biassing means coupled between the housing and the second lock member to bias the second lock member from the primed position towards the locked position, at least one of the first and second lock members being deformable to permit engagement of the first and second lock members, when the second lock member moves from the primed position to the locked position under the action of the biassing means, to lock the lock members to each other.

2. A lock device as claimed in Claim 1 for use with a pneumoperitoneal needle to maintain an obturator in its extended position after the pneumoperitoneal needle has penetrated a body cavity.

3. A lock device as claimed in Claim 1 or Claim 2, wherein the deformable lock member is provided with a deformable flange which engages with a recess in the other lock member.

4. A lock device as claimed in any one of Claims 1, 2 or 3, wherein one of the lock members is deformable and the other of the lock members has a tapered profile which assists the deformation of the deformable lock member.

5. A lock device as claimed in Claim 4, wherein said other lock member has a frustoconical profile.

6. A lock device as claimed in any one of the preceding Claims, wherein the first lock member is deformable.

7. A lock device as claimed in any one of the preceding Claims, wherein the biassing means comprises a helical spring.

8. A lock device as claimed in any one of the preceding Claims, wherein the biassing means requires a compression force of between 1.47 Newtons and 1.57 Newtons to allow movement of the second lock member to the primed position.

9. A lock device as claimed in any one of the preceding Claims, wherein the lock member can be released from engagement with each other by applying a force greater than 4.9 Newtons to one of the lock members to move the second lock member to the unlocked position.

10. A pneumoperitineal device comprising a hollow needle, an obturator mounted within the needle and movable between a position in which it extends beyond the distal tip of the needle and a position in which it is retracted within the needle, the device having a housing at its proximal end, first and second lock members within the housing, the second lock member being movable between an unlocked position, a primed position and an locked position, and biassing means coupled between the housing and the second lock member to bias the second lock member from the primed position towards the locked position, the first and second lock members having thereon engagement means which mutually engage to hold the lock members together and resist mutual disengagement when the second lock member is moved by the biassing means from the primed position to the locked position.

FIG.1

FIG.2

FIG.3

FIG.4

EP 0 519 721 A1

**European Patent Office**

**EUROPEAN SEARCH REPORT**

Application Number

EP 92 30 5605

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| X | EP-A-0 265 193 (USSC) | 1,6,7,10 | A61B17/34 |
| Y | * abstract; figure 2 * | 2-5,10 | |
| | --- | | |
| Y | EP-A-0 405 883 (ROCKET OF LONDON) | 2,10 | |
| | * abstract; figure 2 * | | |
| | --- | | |
| Y | EP-A-0 339 460 (BAUMGART) | 3 | |
| | * column 6, line 52 - line 55; figures 2,3 * | | |
| | --- | | |
| Y | EP-A-0 144 483 (ARZNEIMITTEL) | 4,5 | |
| | * abstract; figure 2 * | | |
| | --- | | |
| A | US-A-4 722 352 (SARSTEDT) | 4,5 | |
| | * abstract; figure 1 * | | |
| | --- | | |
| A | US-A-4 874 382 (LINDEMANN) | | |
| | ----- | | |
| | | | TECHNICAL FIELDS SEARCHED (Int. Cl.5 ) |
| | | | A61B |

The present search report has been drawn up for all claims

| Place of search | Date of completion of the search | Examiner |
|---|---|---|
| THE HAGUE | 28 AUGUST 1992 | BARTON S. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P0401)